# EUROPEAN PATENT APPLICATION

(11) **EP 1 564 216 A1**
(43) Date of publication of application: **17.08.2005**
(21) Application number: 03758859.7
(22) Date of filing: 24.10.2003
(51) Int. Cl.: C07D 471/04, C09K 11/06, H05B 33/14

(54) **WHITE ORGANIC FLUORESCENT COMPOUND**

(30) Priority: 29.10.2002 JP 2002315110
(71) Applicant: HIROSE ENGINEERING CO., LTD., Tokyo 146-0092 (JP)
(72) Inventor: NAKAYA, Tadao, Hirose Engineering Co.,Ltd., Tokyo 146-0092 (JP); IKEDA, Atsushi, Hirose Engineering Co.,Ltd., Tokyo 146-0092 (JP); SUDOH, Hisashi, Hirose Engineering Co.,Ltd., Tokyo 146-0092 (JP)
(74) Representative: Leonhard, Frank Reimund, Dipl.-Ing.
(86) International application number: PCT/JP2003/013598
(87) International publication number: WO 2004/039805

(57) **Abstract**

The objective of the present invention is to provide a fast and weatherable fluorescent compound capable of emitting white light by itself. The compound has the following structure with a quinacridone skeleton.

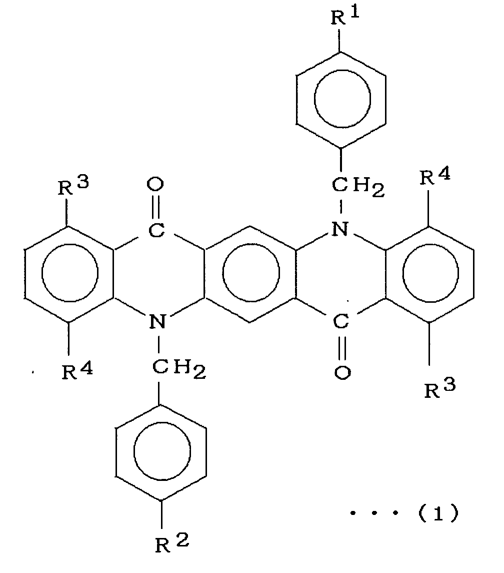

## Description

### Technical Field

The present invention relates to an organic white-fluorescent compound. More particularly, this invention relates to an organic white-fluorescent compound capable of emitting white light at a high luminance, which fluorescent compound has a quinacridone skeleton and therefore is fast and excellent in workability.

### Background Art

Researchers have developed organic EL elements, centering on the development of elements emitting light, the color of which is one of the three primary colors, i.e. red (R), green (G) and blue (B), and that of white light-emitting elements. The emission of white light was realized typically through mixing three compounds respectively emitting red light, blue light and green light, or mixing several light-emitting compounds each having lights of different colors.

However, few compounds that emit white light by themselves are known.

### Summary of the Invention

An objective of the present invention is to provide an organic white-fluorescent compound capable of emitting white light by itself and applicable, for example, to organic EL elements. Another objective of the present invention is to provide an organic fluorescent compound capable of emitting white light and applicable to various kinds of white light illuminators including organic EL elements. As a result of intensive studies to achieve the objectives, the inventors succeeded in synthesizing a single fluorescent compound capable of emitting white light, which led to a long-life EL element emitting a white light of high purity at high luminance. The present invention is in contrast to the conventional technology which provides white-light emission by mixing the colors of the lights emitted by pigments that respectively have three primary colors or by several pigments.

In order to achieve the objectives, the present invention provides an organic white-fluorescent compound having the structure represented by formula (1). 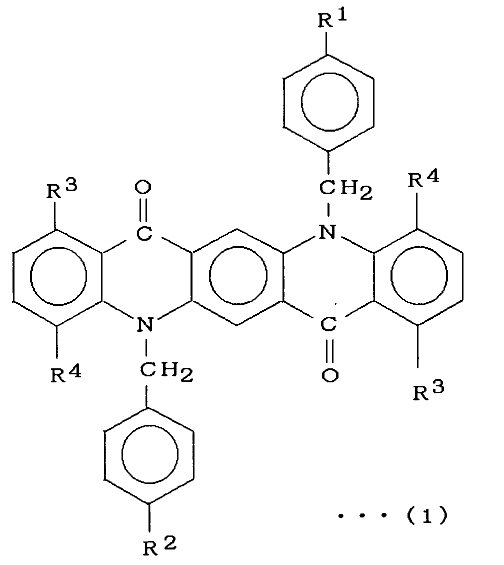 In formula (1), R¹ and R², which may be the same or different from each other, are alkyl groups or alkoxyl groups, and R³ and R⁴, which may be the same or different from each other, are alkyl groups.

Among the compounds represented by formula (1) are preferred organic white-fluorescent compounds in which R¹ and R² are lower alkyl groups having from 1 to 5 carbon atoms, and R³ and R⁴ are lower alkyl groups having from 1 to 5 carbon atoms.

### Brief Description of the Drawings

Figure 1 is an illustration showing an example of the luminescent element according to the present invention.
Figure 2 is an illustration showing another example of the luminescent element according to the present invention.
Figure 3 is an illustration showing a still another example of the luminescent element according to the present invention.
Figure 4 is an illustration showing a further example of the luminescent element according to the present invention.
Figure 5 is an IR spectrum chart of the solid matter represented by formula (10), which was synthesized in Working Example 1.
Figure 6 is an NMR spectrum chart of the solid matter represented by formula (10), which was synthesized in Working Example 1.
Figure 7 is an IR spectrum chart of the solid matter represented by formula (11), which was synthesized in Working Example 1.
Figure 8 is an NMR spectrum chart of the solid matter represented by formula (11), which was synthesized in Working Example 1.
Figure 9 is an IR spectrum chart of the methylbenzylated compound, which was synthesized in Working Example 1.
Figure 10 is an NMR spectrum chart of the methylbenzylated compound, which was synthesized in Working Example 1.
Figure 11 is an IR spectrum chart of the target compound synthesized in Working Example 1.
Figure 12 is an NMR spectrum chart of the target compound synthesized in Working Example 1.
Figure 13 is a fluorescence spectrum chart of the target compound synthesized in Working Example 1.
Figure 14 is an IR spectrum chart of the target compound synthesized in Working Example 2.
Figure 15 is an NMR spectrum chart of the target compound synthesized in Working Example 2.
Figure 16 is a fluorescence spectrum chart of the target compound synthesized in Working Example 2.

### Detailed Description of the Preferred Embodiments

All the organic white-fluorescent compounds represented by formula (1) have the quinacridone skeleton represented by formula (7).

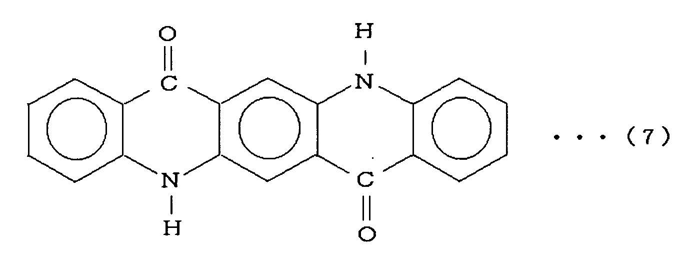

The compound represented by formula (7) is known as a pigment called quinacridone or pigment violet 19. This compoundmaybe synthesized, from, for example, diethyl succinyl succinate and aniline through, for example, condensation, ring closure, and oxidization. Quinacridone itself is excellent in fastness, weatherability, and heat resistance.

Therefore, the organic white-fluorescent compound shown by formula (1) of the present invention is also excellent in fastness, weatherability, and heat resistance.

In formula (1) R¹ and R², which may be the same or different from each other, are alkyl groups or alkoxyl groups. Preferable alkyl groups are lower alkyl groups having from 1 to 5 carbon atoms. Specific examples of R¹ and R², when they are lower alkyl groups, include methyl group, ethyl group, propyl group, butyl group and pentyl group. Also, when R¹ and R² are lower alkoxy groups, specific examples thereof are methoxyl group, ethoxyl group, propoxyl group, butoxyl group and pentoxyl group. Although R¹ and R² may be the same or different from each other, it is preferable if they are the same.

In formula (1), R³ and R⁴, which may be the same or different from each other, are alkyl groups. Preferable examples of R³ and R⁴ are methyl group, ethyl group, propyl group, butyl group and pentyl group.

The organic white-fluorescent compound represented by formula (1) may be prepared in accordance with the Reaction Formulas shown below.

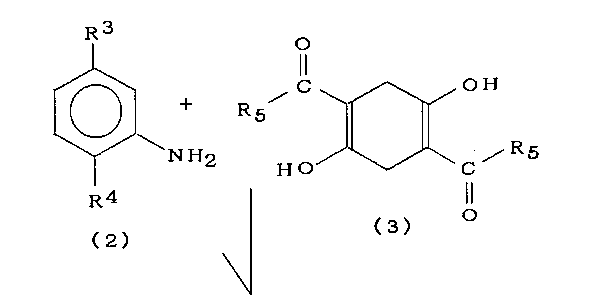

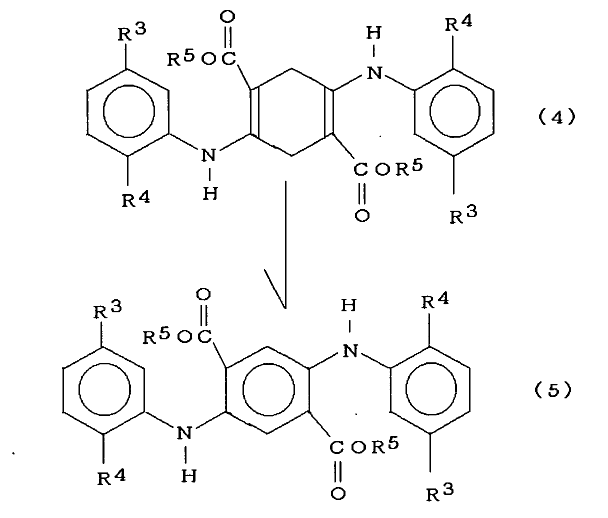

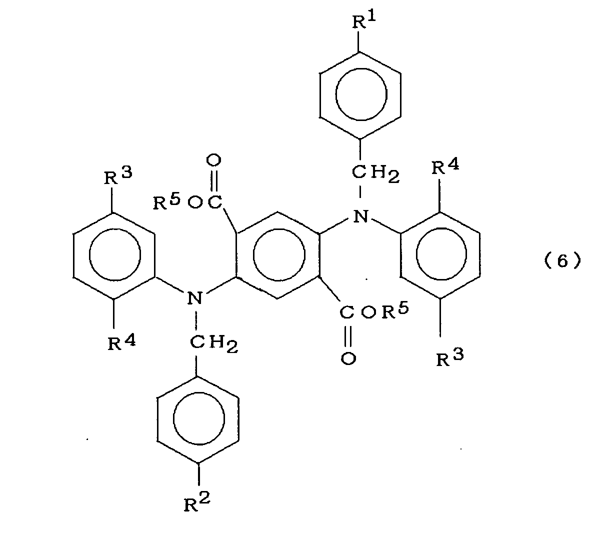

In the Reaction Formulas, R¹, R², R³ and R⁴ are the same as those defined above. R⁵ denotes an alkyl group, preferably an alkyl group having from 1 to 3 carbon atoms.

In the reaction formula above, a diamine represented by formula (4) is obtained through the reaction of 2,5-dialkylaniline represented by formula (2) and 1,4-dimethoxycarbonyl-2,5-dihydroxy-cyclohexadiene represented by formula (3). The reaction can proceed quickly simply by heating the reactants in a solvent such as alcohol in the presence of, for example, acetic acid.

Dehydrogenation of the diamine represented by formula (4) quickly proceeds by heating it in the presence of sulfuric acid, and then it turns to a diaminobenzene represented by formula (5). The heating temperature should be just below the boiling point of the solvent. The reaction time is from 0.5 hour to several hours.

Then, the nitrogen atoms of the diaminobenzene represented by formula (5) are alkylated. The alkylating agent is R¹-C₆H₅-CH₂X or R²-C₆H₅-CH₂X, wherein X denotes a halogen atom such as a chlorine atom. R¹ and R² are the same as those defined above. The alkylating reaction proceeds by heating the diaminobenzene and the alkylating agent in a polar solvent such as DMF at a temperature not higher than the boiling point of the polar solvent.

A ring-closing reaction of the obtained diaminobenzene compound represented by formula (6) is carried out in a polar solvent such as dichlorobenzene in the presence of a dehydrating agent such as p-toluenesulfonic acid by heating. This ring-closing reaction produces the organic white-fluorescent compound having a quinacridone skeleton according to the present invention.

The organic white-fluorescent compound according to the present invention has a special molecular structure: The compound has a quinacridone skeleton including two nitrogen atoms, each of which is bonded to an alkyl- or alkoxyl-benzyl group. The quinacridone skeleton has two heterocyclic rings, each of which includes one of the two nitrogen atoms and a carbonyl group. The quinacridone skeleton also has two terminal benzene rings, each of which is located to the outside of each heterocyclic ring on the opposite side of the central benzene ring. Furthermore, two alkyl groups are bonded to each terminal benzene ring. The alkyl- or alkoxyl-benzyl groups respectively bonded to the nitrogen atoms cause the emission of red light in this organic white-fluorescent compound. On the other hand, two alkyl groups bonded to each terminal benzene ring cause the emission of blue light. The alkyl groups with larger molecular weights lead to a more intense emission. Also, the quinacridone skeleton causes the emission of green light. The green light emission resulting from the quinacridone skeleton, the blue light emission resulting from the two alkyl groups bonded to each terminal benzene ring, and the red light emission resulting from the alkyl- or alkoxyl-groups bonded to the nitrogen atoms are caused simultaneously by the application of electromagnetic wave energy from the outside. As a result, the human eye recognizes the mixed lights as white light.

The organic white-fluorescent compound according to the present invention has the fluorescence peaks of blue, green and red in the wavelength range between 400 nm and 650 nm. This property enables the compound to be utilized for an organic electroluminescent element that is capable of emitting white light.

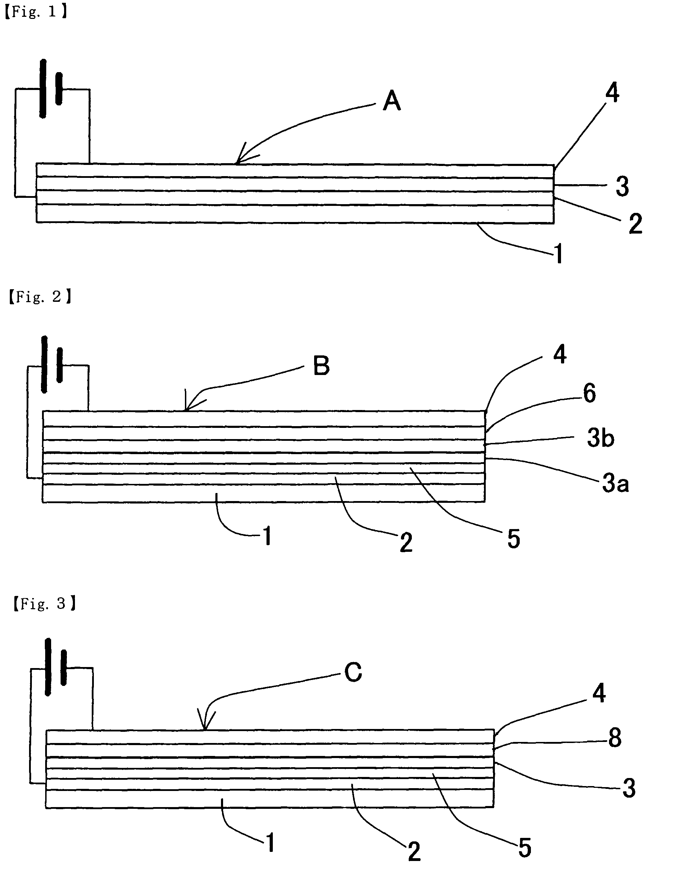

Figure 1 is a schematic illustration that shows the sectional structure of a luminescent element, which is a one-layer type organic EL element. As shown in this figure, a luminescent element A is prepared by layering a light-emitting layer 3 and an electrode layer 4 in this order on a substrate 1 with which a transparent electrode 2 has been provided. The light-emitting layer 3 includes the organic white-fluorescent compound according to the present invention.

When electric current is applied to the luminescent element A shown in Figure 1 at the transparent electrode 2 and the electrode layer 4, the element emits white light due to the organic white-fluorescent compound. Upon the application of an electric field between the transparent electrode 2 and the electrode layer 4, electrons are injected from the electrode layer 4 and positive holes from the transparent electrode 2. In the light-emitting layer 3, the electrons are recombined with positive holes, which causes the energy level to return to the valence band from the conduction band. This transition of the energy level is accompanied by emission of the energy differential as light.

The luminescent element A shown in Figure 1, when it is shaped to a planar form with a large area, may be used as a planar illuminator, for example a large-area wall illuminator when fixed on a wall, or a large-area ceiling illuminator when fixed on a ceiling. This luminescent element may be utilized for a planar light source in place of a point light source, such as a conventional bulb, and a line light source, such as a conventional fluorescent lamp. In particular, this illuminator can suitably be used to light up walls, ceilings and floors in dwelling rooms, offices and passenger trains, or to make them emit light. Moreover, this luminescent element A may be suitable for the backlight used in displays of computers, cellular phones and ATMs. Furthermore, this illuminator may be used for various light sources, such as the light source of direct illumination and that of indirect illumination. Also, it may be used for the light sources of advertisement apparatuses, road traffic sign apparatuses and light-emitting billboards, which have to emit light at night and provide good visibility. In addition, because this luminescent element A includes the organic white-fluorescent compound in the light-emitting layer, the luminescent element A may have a long life. Therefore, light sources employing the luminescent element A will naturally have a long life.

The luminescent element Amay also be shaped into a tubular light emitter comprising a tubularly shaped substrate 1, a transparent electrode 2 placed on the inside surface of the substrate 1, a light emitting layer 3 and an electrode layer 4 placed on the transparent electrode 2 in this order. Because the luminescent element A does not include mercury, it is an ecological light source and may be a substitute for conventional fluorescent lamps.

For the substrate 1 may be used any known substrate, as long as the transparent electrode 2 can be formed on the surface of the substrate. Examples of the substrate 1 are a glass substrate, a plastic sheet, a ceramic substrate, and a metal substrate of which surface is insulated, for example, through the formation of an insulating layer thereon.

The light-emitting layer 3 may be in the shape of a film or a sheet including the organic white-fluorescent compound according to the present invention.

The thickness of the light-emitting layer 3 ranges, typically between 30 nm and 500 nm, preferably between 100 nm and 300 nm. When the thickness is too small, the amount of the emitted light may be insufficient. On the other hand, when the thickness is too large, the voltage required to drive the illuminator or element may be too high, which is not desirable. Besides, the large thickness may reduce the flexibility of the film necessary to shape a planar, tubular, curved, or ring article.

The film or sheet including the organic white-fluorescent compound may be formed through the application of a solution of the organic white-fluorescent compound dissolved in a suitable solvent. The application method is one selected from a spin cast method, a coating method, a dipping method, etc. Also, the light-emitting layer 3 may be made through sandwiching a powder of the organic white-fluorescent compound between the electrodes, melting the powder, and contact-bonding the melted powder.

For the electrode layer 4 may be employed a material having a small work function. Examples of the material are elementary metals and metallic alloys, such as MgAg, aluminum alloy, metallic calcium, etc. A preferable electrode layer 4 is made of an alloy of aluminum and a small amount of lithium. This electrode 4 may easily be formed on the surface of light-emitting layer 3, which, in turn, has been formed on substrate 1, by the technique of metal deposition.

When either of the application method or the melting and contact-bonding method is employed, a buffer layer should be inserted between each electrode and the light-emitting layer.

Materials for the buffer layer are, for example, an alkaline metal compound such as lithium fluoride, an alkaline earth metal compound such as magnesium fluoride, an oxide such as an aluminum oxide, and 4, 4'-biscarbazole biphenyl (Cz-TPD) . Also, materials for forming the buffer layer between the cathode made of a material such as ITO and the organic layer are, for example, m-MTDATA (4,4',4"-tris(3-methylphenyl-phenylamino) triphenylamine), phthalocyanine, polyaniline, and polythiophene derivatives, and inorganic oxides such as molybdenum oxide, ruthenium oxide, vanadium oxide and lithium fluoride. When the materials are suitably selected, these buffer layers can lower the driving voltage of the organic EL element, which is the luminescent element, improve the quantum efficiency of luminescence, and achieve an increase in the luminance of the emitted light.

The second example of the luminescent element according to the present invention is shown in Figure 2. This figure is an illustration showing the sectional layer structure of an example of the luminescent element, which is a multi-layer organic EL element.

As shown in Figure 2, the luminescent element B comprises a substrate 1, and a transparent electrode 2, a hole-transporting layer 5, light-emitting sublayers 3a and 3b, an electron-transporting layer 6, and an electrode layer 4, the layers being laid on the substrate 1 one by one in this order.

The substrate 1, the transparent electrode 2 and the electrode layer 4 are the same as those explained for the luminescent element A in Figure 1.

The light-emitting layer of the luminescent element B comprises light-emitting sublayers 3a and 3b. The light-emitting sublayer 3a is a deposited film formed by depositing the organic white-fluorescent compound on the hole-transporting layer 5. The light-emitting sublayer 3b is a DPVBi layer, which functions as a host material.

Examples of the hole-transporting substance included in the hole-transporting layer 5 are a triphenylamine compound such as N,N'-diphenyl-N,N'-di(m-tolyl)-benzidine (TPD) and α -NPD, a hydrazon compound, a stilbene compound, a heterocyclic compound, a π electron star burst positive hole transporting substance, etc.

Examples of the electron-transporting substance included in the electron-transporting layer 6 are an oxadiazole derivative such as 2-(4-tert-butylphenyl)-5-(4-biphenylyl)-1,3,4-oxadiazole and 2,5-bis(1-naphthyl)-1,3,4-oxadiazole (BND), and 2,5-bis(5'-tert-butyl-2'-benzoxazolyl) thiophene. Also, a metal complex material such as quinolinol aluminum complex (Alq3), benzoquinolinol beryllium complex (Bebq2) may be used suitably.

The electron-transporting layer 6 of the luminescent element B shown in Figure 2 includes Alq3 as electron-transporting substance.

The thickness of each layer is the same as that in a known multi-layer organic EL element.

The luminescent element B in Figure 2 functions and emits light in the same ways as the luminescent element A in Figure 1. Therefore, the luminescent element B has the same uses as the luminescent element A.

The third example of the luminescent element of the present invention is shown in Figure 3. This figure is an illustration showing the sectional layer structure of an example of the luminescent element, which is a multi-layer organic EL element.

The luminescent element emitting white light C shown in Figure 3 comprises a substrate 1, and a transparent electrode 2, a hole-transporting layer 5, a light-emitting layer 3, an electron- transporting layer 8, and an electrode layer 4, wherein the transparent electrode and the layers are laid on substrate 1 one by one in this order.

The luminescent element C functions in the same way as the luminescent element B.

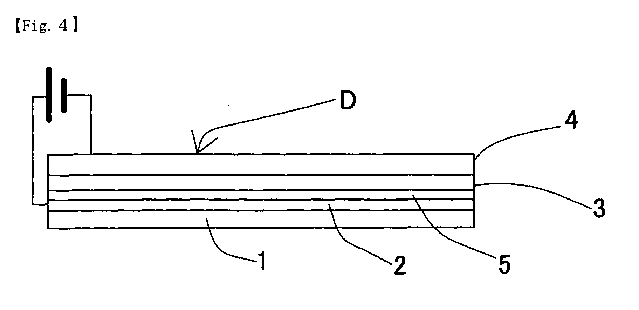

Another example of the luminescent element of this invention is shown in Figure 4. The luminescent element D comprises a substrate 1, and a transparent electrode 2, a hole-transporting layer 5, a light-emitting layer 3, and an electrode layer 4 wherein the transparent electrode and the layers are laid on the substrate 1 one by one in this order.

An example of the luminescent elements, other than those shown in Figures 1-4, is a two-layer lowmolecular weight organic luminescent element having a hole-transporting layer that includes a hole-transporting substance and an electron-transporting light-emitting layer that includes the organic white-fluorescent compound of the invention laid on the hole-transporting layer, these layers being sandwiched between a cathode, which is the transparent electrode formed on the substrate, and an anode, which is the electrode layer. A specific example of this embodiment is a two-layer pigment-injected luminescent element comprising a hole-transporting layer and a light-emitting layer that includes a host pigment and the organic white-fluorescent compound of the present invention as a guest pigment, wherein the light-emitting layer is laid on the hole-transporting layer and these layers are sandwiched between the cathode and the anode. Another example is a two-layer organic luminescent element comprising a hole-transporting layer that includes a hole-transporting substance and an electron-transporting light-emitting layer that is made of the organic white-fluorescent compound of the invention and an electron-transporting substance, the latter layer being laid on the former, and these two layers being sandwiched between the cathode and the anode. A specific example of the second embodiment is a two-layer pigment-injected luminescent element comprising a hole-transporting layer and an electron-transporting light-emitting layer that includes a host pigment and the organic white-fluorescent compound of the present invention as a guest pigment, wherein the light-emitting layer is laid on the hole-transporting layer and these layers are sandwiched between the cathode and the anode. A further example is a three-layer organic luminescent element comprising a hole-transporting layer, a light-emitting layer including the organic white-fluorescent compound of the present invention that is laid on the hole-transporting layer, and an electron-transporting layer that is laid on the light-emitting layer, these layers being sandwiched between the cathode and the anode.

Also, it is preferable, if the light-emitting layer includes, as a sensitizing agent, rubrene, especially both of rubrene and Alq3.

An organic EL element utilizing the organic white-fluorescent compound of the present invention may generally be used as an organic EL element which is driven, generally, by direct current, and also by pulses and alternating current.

The organic white-fluorescent compound according to the present invention may also be used in the field of displays such as black-and-white displays and color displays, and in the field of illumination such as lighted signs and advertisements, signs and advertisements made by light, indirect illumination, or backlight for LCDs.

### Examples

### (Working Example 1)

A mixture of 20 g of 2,5-dimethylaniline and 17.12 g of 1,4-dimethoxy-carbonylcyclohexadiene, represented by formula (9), in a mixed solvent of 300 ml of acetic acid and 300 ml of ethanol were allowed to react at 115°C with stirring for 24 hours. After the termination of the reaction, the obtained mixture was cooled with cold water. The cooled mixture was filtered with a glass filter. The obtained solids were washed with methanol, ethyl acetate, THF and petroleum ether in this order. The washed was dried under vacuum for about two hours. 19.46 g of a yellowish orange solid matter was obtained.

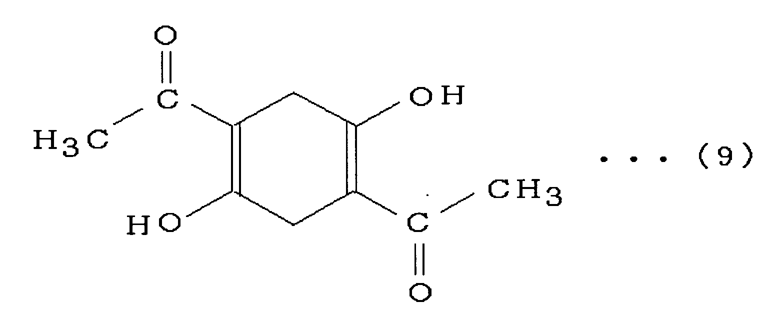

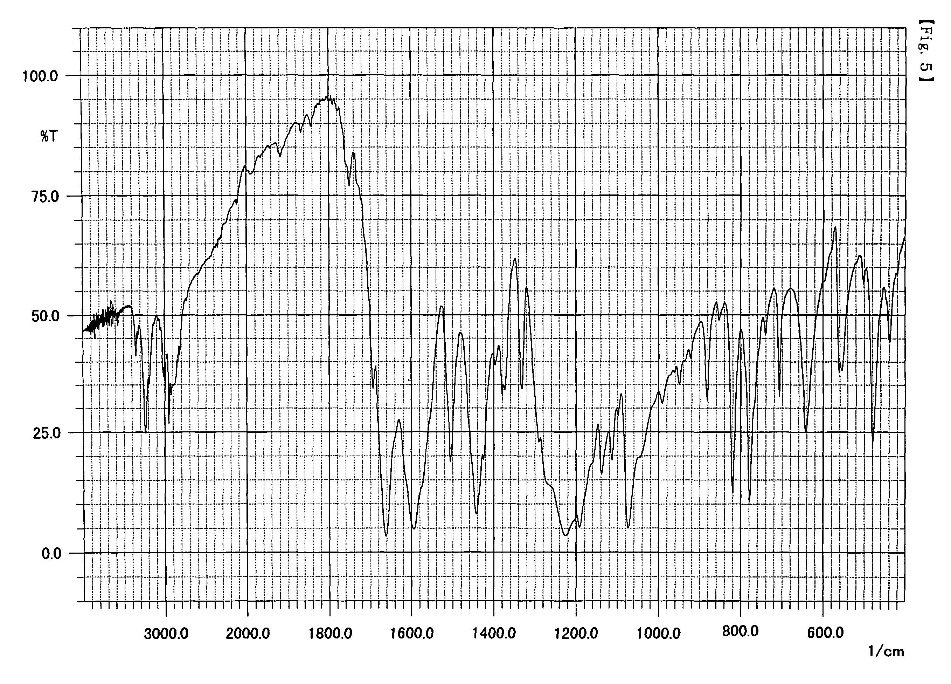

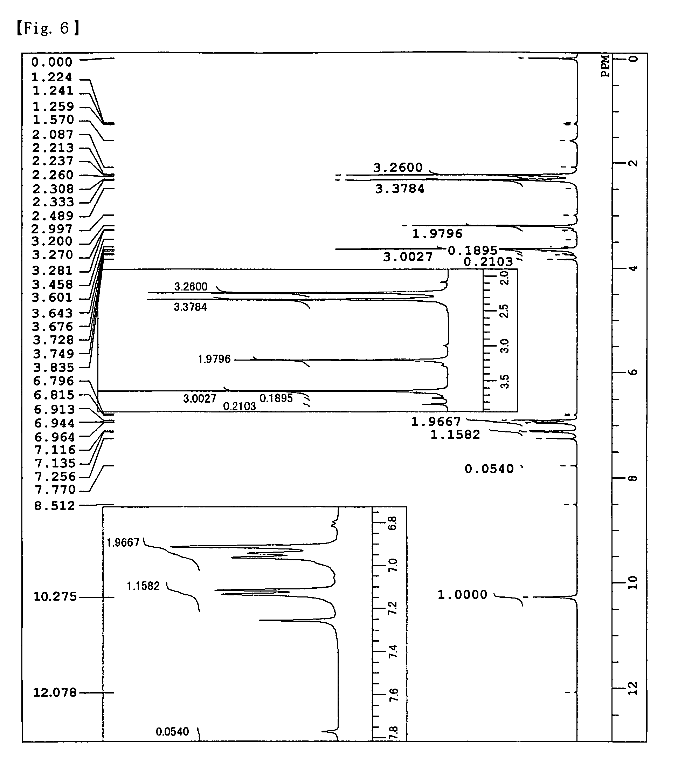

An NMR spectrum chart and an IR spectrum chart of the solid matter are respectively shown in Figure 5 and Figure 6. This solid matter was identified as a compound having the structure represented by formula (10) below.

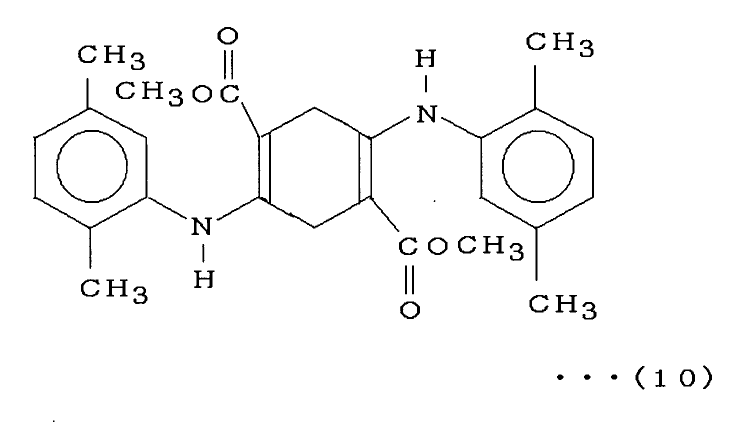

Next, 19.46 g of the compound represented by formula (10) in o-dichlorobenzen was stirred for an hour at 160°C in the presence of 20 drops of sulfuric acid to be subjected to a dehydrogenation reaction. After the reaction, the obtained solution was cooled with cold water, and filtered with a glass filter. The obtained solids were washed with methanol and petroleum ether, and then dried. 12.36 g of an orange solid matter was obtained.

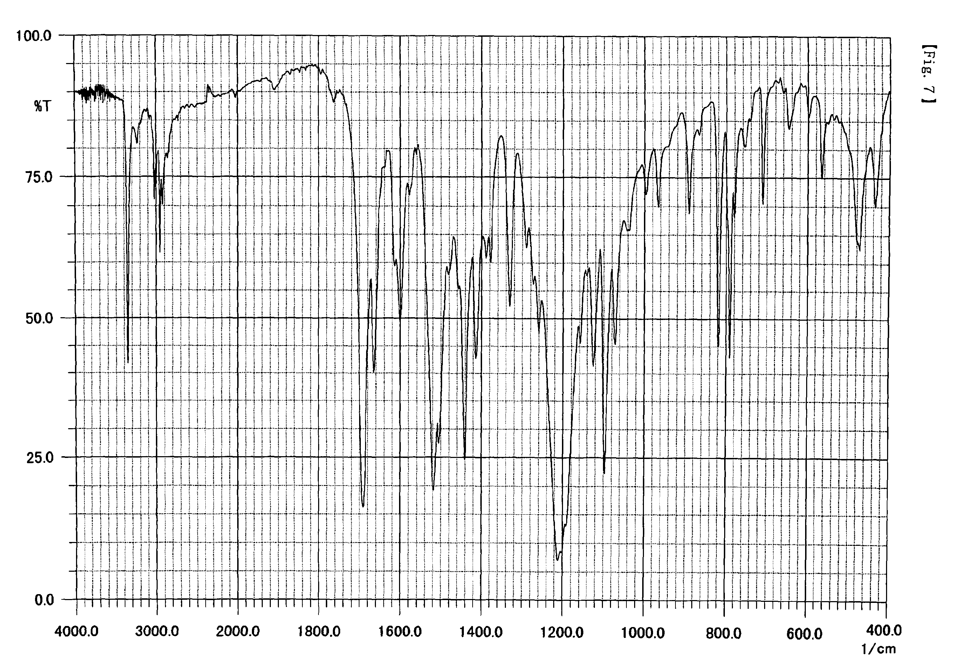

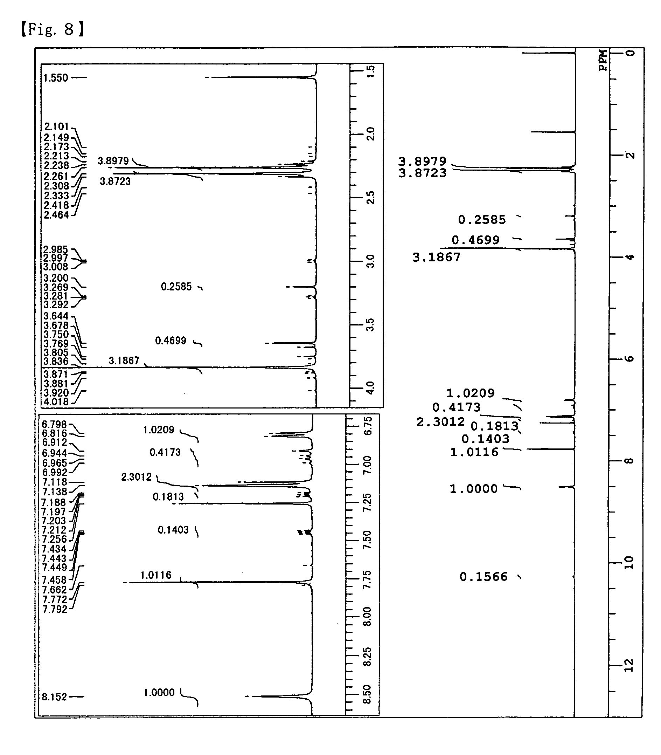

An NMR spectrum chart and an IR spectrum chart of the solid matter are respectively shown in Figure 7 and Figure 8. This solid matter was identified as a compound having the structure represented by formula (11) below.

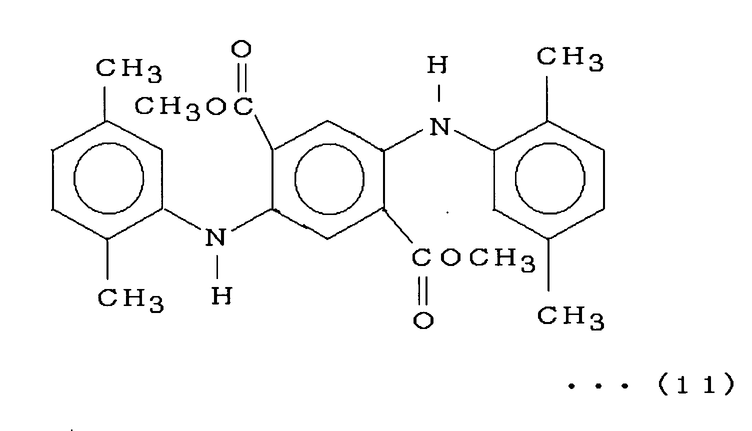

3 g of the compound represented by formula (11) and 5.9 g of 4-chloromethyltoluene in a mixed solvent of 200 ml of DMF and 5.9 g of α-chloroxylene were stirred for 2 hours at 160°C. The product obtained through the reaction was allowed to stand for 2 days and naturally cooled. Then the product was heated to a temperature of 60 to 70°C and the solvent was evaporated away with an evaporator. Solids were obtained. The solids were mixed with 200 ml of chloroform and 200 ml of water, and the chloroform phase was separated from the water phase. The separated chloroform solution was neutralized by a 30% aqueous solution of caustic soda. The neutralized solution was washed with water twice, and moisture in the solution was removed with Glauber's salt. Then, the Glauber's salt was filtered out. After the solvent was removed, solids were separated. The solids were washed with petroleum ether and dried. 0.45 g of a light orange solid matter was obtained.

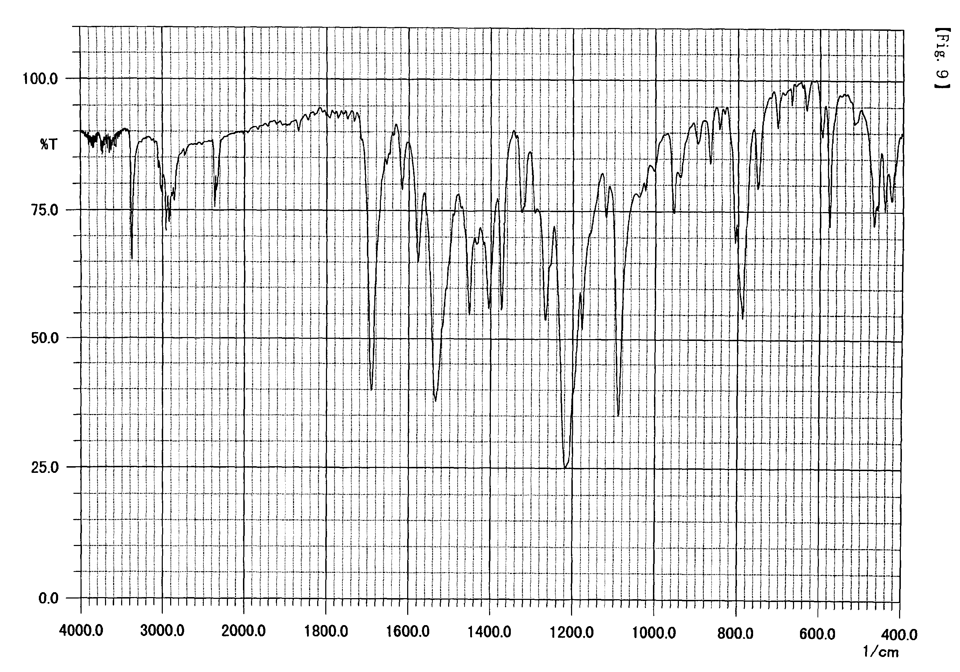

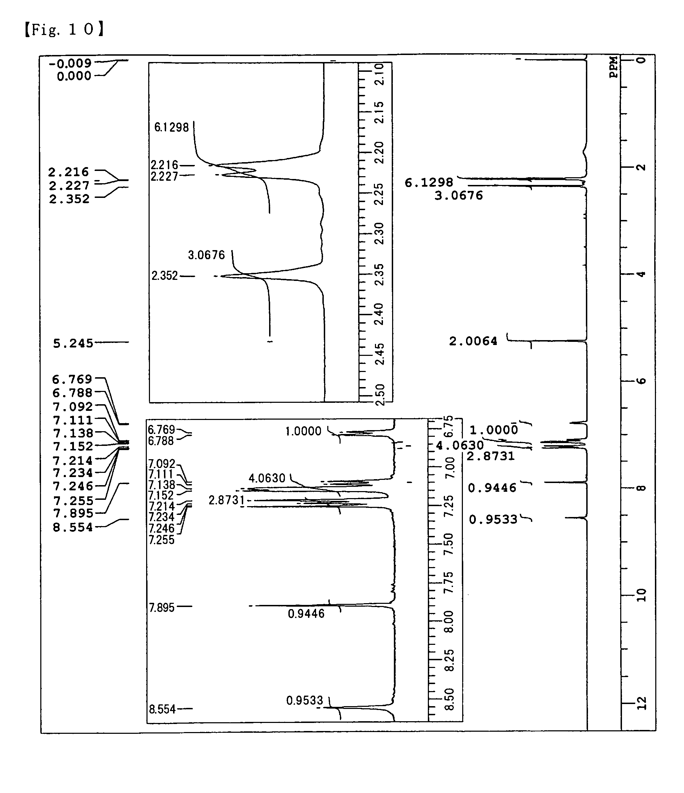

From an NMR spectrum chart and an IR spectrum chart of the solid matter shown in Figure 9 and Figure 10 respectively, this solid matter was identified as a compound having the structure represented by formula (12) below.

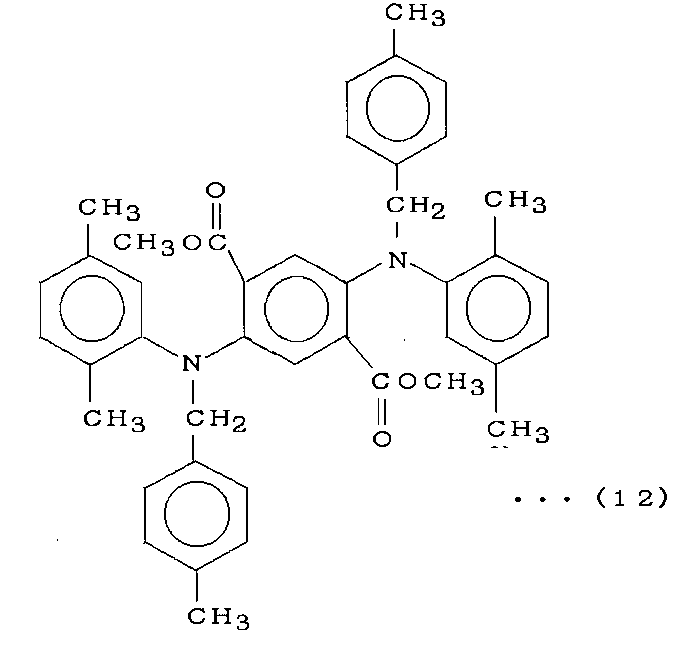

0.45 g of the methylbenzylated compound represented by formula (12) in 100 ml of o-dichlorobenzene in the presence of 0.80 g of p-toluenesulfonic acid were stirred for 20 hours at 160°C. After the termination of the reaction, the obtained solution was cooled naturally. Then the solvent was evaporated away from the cooled solution. Solids remained. The solids were washed with methanol, and then with petroleum ether. 0.05 g of black solid matter was obtained.

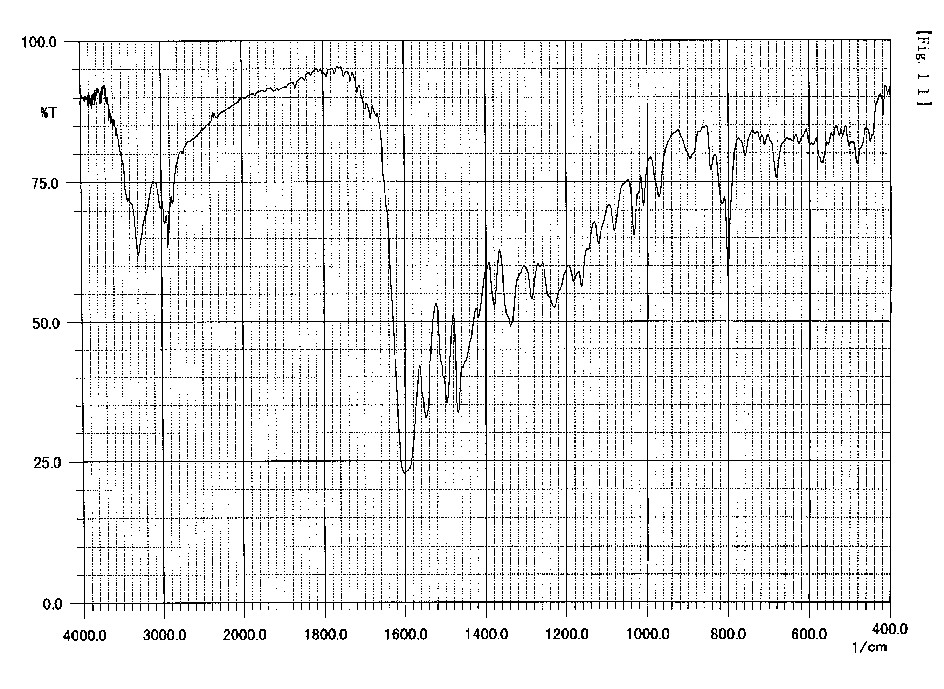

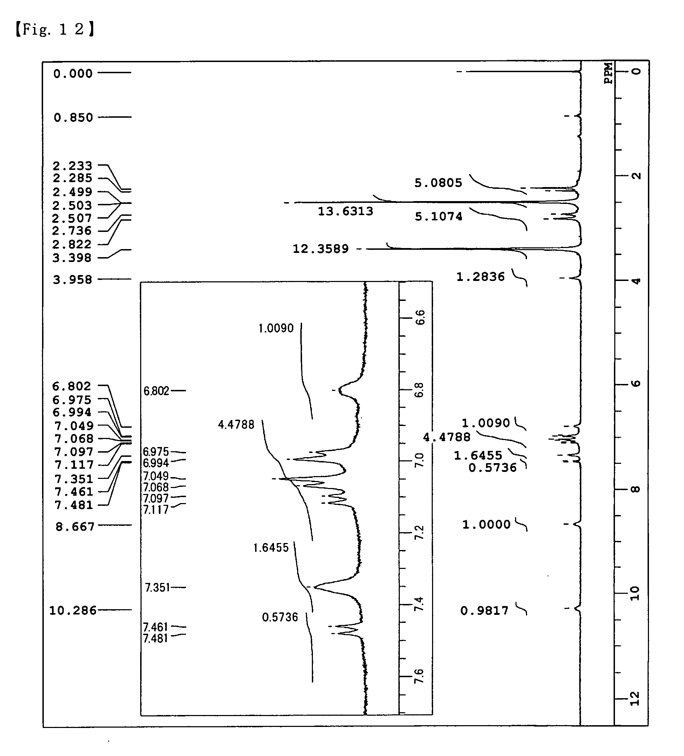

From an NMR spectrum chart and an IR spectrum chart of the solid matter shown in Figure 11 and Figure 12 respectively, this solidmatter was identified as a target compound represented by formula (13).

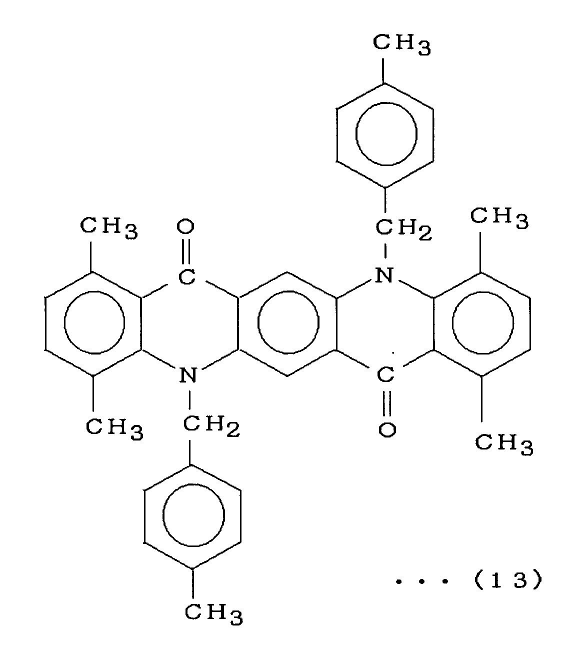

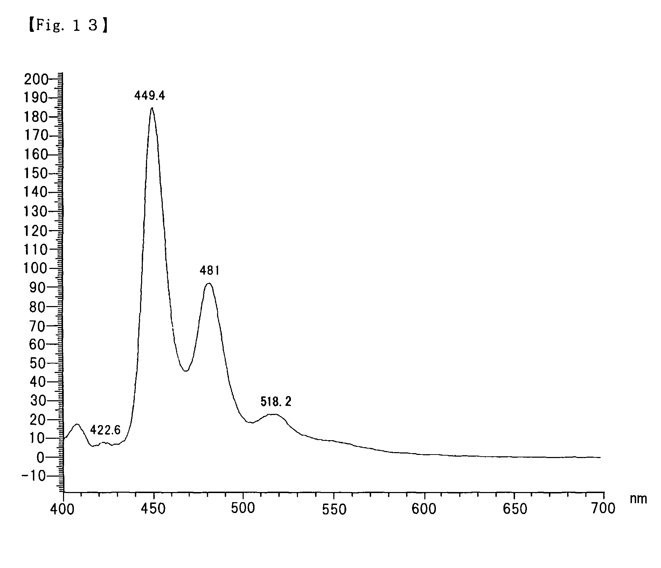

A sample solution was prepared by dissolving the target compound represented by formula (13) in xylene so that the concentration of the target compound was 10 mg/L. This sample solution was loaded in a model F-4500 spectrofluorophotometer, a product by Shimadzu Corporation, and the fluorescence spectrum of the solution was measured under the following conditions. The measured spectrum is shown in Figure 13.

### Conditions of Measurement

Measuring mode: Wavelength scanning
Exciting wavelength: 365 nm
Wavelength at which the emission of fluorescence started: 400 nm
Wavelength at which the emission of fluorescence ended: 700 nm
Scanning speed: 240 nm/min.
Slit on the side of excitation: 5.0 nm
Slit on the side of fluorescence emission: 5.0 nm
Photomal voltage: 700 V

As understood from Figure 13, the target compound obtained in this working example has a fluorescence emission from 400 nm to 600 nm. Specifically, the spectrum has a peak at 516.2 nm, which means an emission of red light. This peak has a long tail extending on the side of longer wavelengths. The spectrum also has the second peak at 481 nm, which means an emission of green light, and the third peak at 449.4 nm, which an means emission of blue light. Thus, the green and blue lights resulting from the second and third peaks as well as the red light resulting from the first peak with the long train on the side of longer wavelengths made the emitted light as a whole white.

### (Working Example 2)

6 g of the compound represented by formula (11) and 13.06 g of 4-methoxybenzyl chloride were reacted with stirring in 300 ml of DMF for 20 hours at 160°C. The product obtained through the reaction was naturally cooled to the room temperature. Then the product was heated to a temperature of 60 to 90°C and the solvent was evaporated away with an evaporator. A viscous substance was obtained. The viscous substance was introduced into methanol. The obtained mixture was filtered and non-dissolved residue was removed. Then, methanol was evaporated away. The remaining was extracted with chloroform, and the extract was neutralized by a 30% aqueous solution of caustic soda. The neutralized extract was washed with water twice, and moisture in the solution was removed with Glauber's salt. Then, the Glauber's salt was filtered out. After the solvent was removed, solids were separated. The solids were washed with petroleum ether, filtered, and dried. 0.87 g of an ocherous solid matter was obtained. This ocherous solid matter was a methoxybenzylated compound having the structure represented by formula (14).

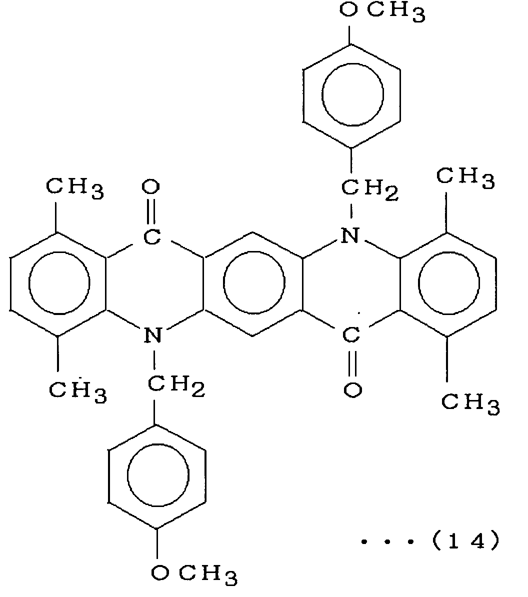

0.87 g of the methylbenzylated compound represented by formula (14) in 150 ml of o-dichlorobenzene in the presence of 1.48 g of p-toluenesulfonic acid were stirred for 20 hours at 160°C. After the termination of the reaction, the obtained solution was cooled naturally. Then the solvent was evaporated away from the cooled solution. Solids remained. The solids were washed with methanol, and then with petroleum ether. 0.23 g of reddish black solid matter was obtained.

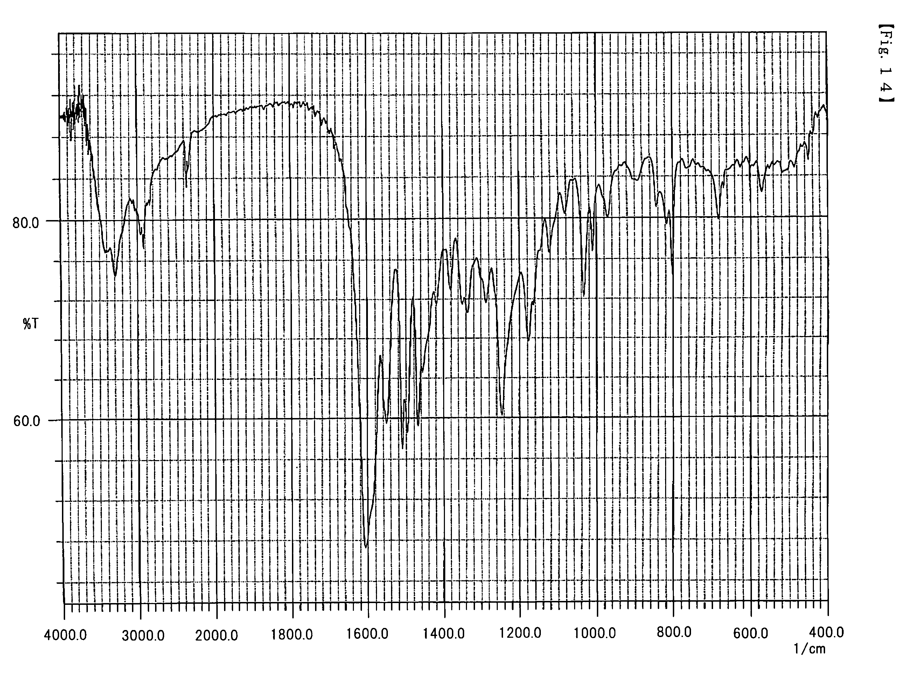

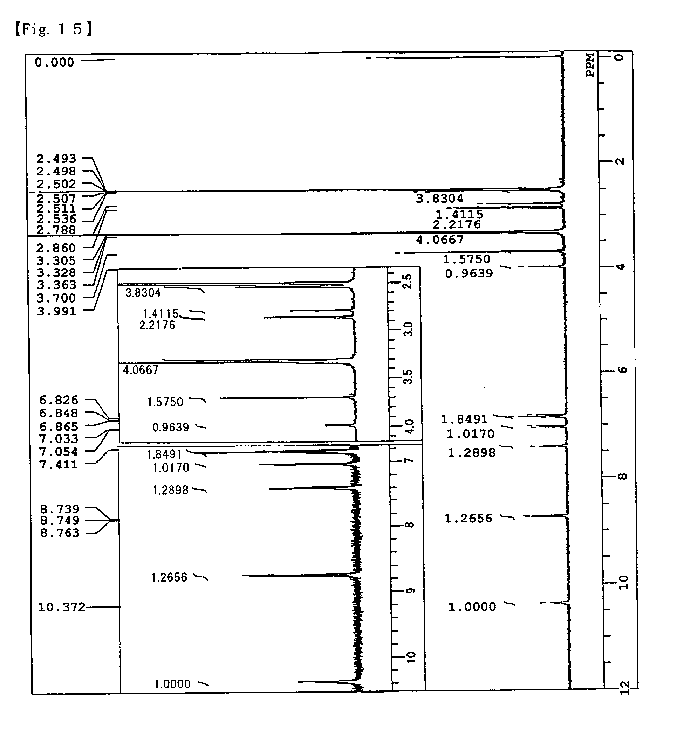

From an NMR spectrum chart and an IR spectrum chart of the solid matter shown in Figure 14 and Figure 15 respectively, this solidmatter was identified as a target compound represented by formula (15).

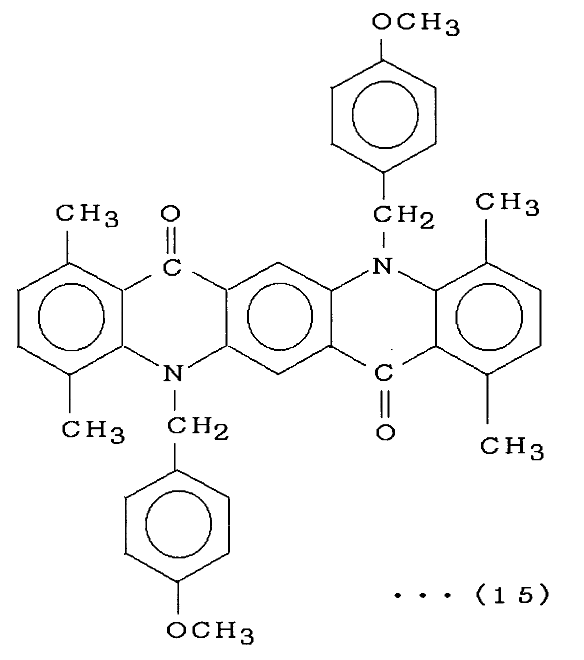

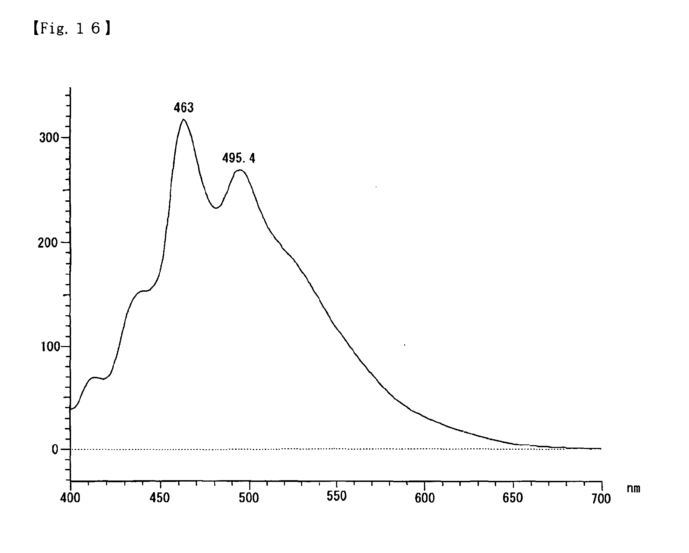

A sample solution was prepared by dissolving the target compound represented by formula (15) in xylene so that the concentration of the target compound was 10 mg/L. This sample solution was loaded in a model F-4500 spectrofluorophotometer, a product by Shimadzu Corporation, and the fluorescence spectrum of the solution was measured under the following conditions. The measured spectrum is shown in Figure 16.

### Conditions of Measurement

Measuring mode: Wavelength scanning
Exciting wavelength: 365 nm
Wavelength at which the emission of fluorescence started: 400 nm
Wavelength at which the emission of fluorescence ended: 700 nm
Scanning speed: 2400 nm/min.
Slit on the side of excitation: 5.0 nm
Slit on the side of fluorescence emission: 5.0 nm
Photomal voltage: 700 V

As understood from Figure 16, the target compound obtained in this working example had a fluorescence emission from 400 nm to 650 nm. Specifically, the spectrum had a wide peak with a ridge at 495.4 nm and a ridgeline extending to 650 nm. The spectrum also had another peak at 463 nm, which means an emission of blue light. Thus, the color of the emitted light was white as a whole.

### Industrial Applicability

The present invention provides an organic white-fluorescent compound capable of emitting white fluorescent light by itself. The wavelength of the emitted light ranges from 400 nm to 650 nm. This organic white-fluorescent compound may be used to make organic EL elements, displays, illuminators and the like emit white fluorescent light.

Also, the employment of a prism that separates the emitted light into blue light, red light and green light would enable the use of this organic white-fluorescent compound as a luminescent element capable of emitting blue light, red light and green light. If a color filter is further employed, the organic white-fluorescent compound may provide a full color display, which can be used for the backlight of LCDs.

## Claims

1. An organic white-fluorescent compound represented by formula (1) : 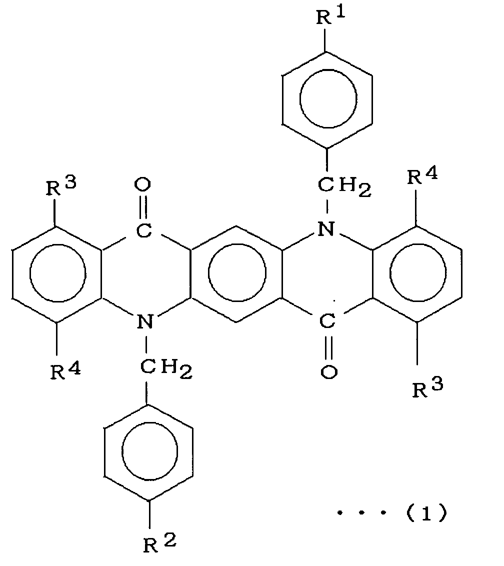 wherein R¹ and R², which may be the same or different from each other, are alkyl groups or alkoxyl groups, and R³ and R⁴, which may be the same or different from each other, are alkyl groups.

2. The organic white-fluorescent compound according to claim 1, wherein each of said R¹ and R² is a lower alkyl group having from 1 to 5 carbon atoms, and each of said R³ and R⁴ is a lower alkyl group having from 1 to 5 carbon atoms.
